# DEMANDE DE BREVET EUROPEEN

(11) **EP 4 785 948 A1**
(43) Date de publication de la demande: **05.08.2026**
(21) Numéro de dépôt: 26154576.8
(22) Date de dépôt: 27.01.2026
(51) Int. Cl.: A61K 36/185, A61P 29/00, A61K 9/14, A61K 36/02

(54) **PRÉPARATION DE COMBRETUM MICRANTHUM G. DON POUR LA PRÉVENTION OU LE TRAITEMENT DE LA DOULEUR**

(30) Priorité: 29.01.2025 FR 2500924
(71) Demandeur: Larena, 49270 Oree D'Anjou (FR)
(72) Inventeur: LEBLANC, Anne, 49110 MONTREVAULT SUR EVRE (FR); BARDOT, Valérie, 03140 TARGET (FR); DUBOURDEAUX, Michel, 03140 TAXAT (FR)
(74) Mandataire: Jacobacci & Partners France

(57) **Abrégé**

La présente invention concerne une préparation de *Combretum micranthum* G. Don, pour son utilisation chez des sujets humains ou animaux, dans le traitement et/ou la prévention de la douleur.

Cette préparation comprend :
- au moins une poudre de feuilles de *Combretum micranthum* G. Don, et
- au moins un extrait aqueux obtenu à partir de feuilles de *Combretum micranthum G.* Don, avantageusement un extrait aqueux concentré.

## Description

### Domaine technique de l'invention

La présente invention concerne le domaine technique des préparations de plantes, en particulier les extraits de plantes, pour leur utilisation chez des sujets humains ou animaux, dans le traitement et/ou la prévention de la douleur.

### Etat de la technique

Actuellement, la prise en charge médicamenteuse des états douloureux chroniques présente des limites.

Le mésusage des médicaments liés à la douleur est un problème très important. Ces mésusages concernent les antalgiques les plus référencés tels que le paracétamol, l'aspirine, les anti-inflammatoires ou la morphine et ses dérivés.

Les antalgiques sont considérés comme actifs et efficaces contre les douleurs aiguës mais présentent des effets secondaires significatifs s'ils sont utilisés de façon prolongée : troubles gastriques, rénaux, dépendance, accoutumance, etc.

Ainsi ces médicaments ne sont pas dénués d'effets indésirables et ne sont donc pas adaptés à une prise chronique ou à la prise en charge des douleurs chroniques.

Pour ces dernières, notamment les douleurs de type neuropathiques, les traitements sont des anti-dépresseurs et des antiépileptiques, qui ont moins d'effets indésirables mais avec une efficacité observable chez seulement 50% des patients.

Il existe ainsi un besoin de développer des traitements efficaces, sûrs et bien tolérés pour les patients.

Dans ce contexte, beaucoup de consommateurs se tournent vers des approches naturelles à base de plantes ou de préparations de plantes pour améliorer leur qualité de vie.

Les préparations de plantes offrent en effet un potentiel pour combler ce besoin en raison de leur efficacité, de leur faible toxicité et de leur disponibilité.

En conséquence, il y a un intérêt continu pour développer de nouvelles préparations de plantes adaptées au traitement et à la prévention de la douleur.

### Présentation de l'invention

Afin de remédier à l'inconvénient précité de l'état de la technique, la présente invention propose une préparation de *Combretum micranthum* G. Don, pour son utilisation chez des sujets humains ou animaux, dans le traitement et/ou la prévention de la douleur.

Cette préparation comprend :
- au moins une poudre de feuilles de *Combretum micranthum* G. Don, et
- au moins un extrait aqueux obtenu à partir de feuilles de *Combretum micranthum* G. Don, avantageusement un extrait aqueux concentré.

En effet, de manière surprenante, la présente invention propose un ingrédient végétal, issu de *Combretum micranthum* G Don, pour traiter ou prévenir la douleur chez les sujets humains ou animaux, avec un profil d'efficacité intéressant.

De plus, cet ingrédient végétal selon l'invention est avantageusement standardisé en termes de composition phytochimique et d'activité biologique.

Un tel ingrédient végétal constitue en outre une ressource naturelle composée d'un mélange de molécules actives (versus une molécule synthétique seule), adaptée à la prise chronique.

Contrairement aux molécules synthétiques généralement utilisées dans la gestion de la douleur, cet ingrédient végétal est composé d'un mélange de molécules actives qui agissent de manière naturelle et complémentaire. Cette composition plurielle permet une adaptation aux traitements chroniques en limitant les effets secondaires souvent observés avec les analgésiques et anti-inflammatoires classiques. De plus, le profil naturel de l'ingrédient en fait une alternative alignée avec les attentes des consommateurs.

Sans être limité par une quelconque théorie, l'efficacité biologique de l'invention est assurée par une action synergique sur l'inhibition des enzymes cyclooxygénase-2 (COX-2) et 5-lipoxygénase (5-LOX), toutes deux impliquées dans les mécanismes inflammatoires et douloureux. Cette double inhibition offre un effet anti-inflammatoire et analgésique renforcé, permettant une prise en charge globale des douleurs inflammatoires.

D'autres caractéristiques non limitatives et avantageuses du produit/procédé conforme à l'invention, prises individuellement ou selon toutes les combinaisons techniquement possibles, sont les suivantes :
- ledit au moins un extrait aqueux obtenu à partir de feuilles de *Combretum micranthum* G. Don est imprégné sur ladite au moins une poudre de feuilles de *Combretum micranthum* G. Don ; de préférence, ladite préparation se présente sous forme d'une poudre imprégnée présentant les caractéristiques granulométriques suivantes : une taille inférieure, ou égale, à 50 µm représentant au maximum 10%, en poids, une taille inférieure, ou égale, à 100 µm représentant au maximum 25%, en poids, et une taille inférieure, ou égale, à 500µm représentant au minimum 90%, en poids ;
- ladite préparation est issue d'un procédé comprenant les étapes suivantes : une étape de contact entre des feuilles de *Combretum micranthum* G. Don et un solvant aqueux, au moins une étape d'extraction en phase liquide des principes actifs, avantageusement réalisée sous chauffage, par exemple à une température allant de 75°C à 90°C, de préférence pendant 0,5 à 2 h, et/ou sous pression réduite, pour obtenir ledit extrait aqueux contenant lesdits principes actifs, de préférence, une étape de concentration dudit extrait aqueux pour obtenir un extrait aqueux concentré, et une étape de dépôt dudit au moins un extrait aqueux obtenu à partir de feuilles de *Combretum micranthum* G. Don sur ladite au moins une poudre de feuilles de *Combretum micranthum* G. Don ; de préférence, ladite étape de dépôt consiste en une étape de séchage dudit au moins un extrait aqueux obtenu à partir de feuilles de *Combretum micranthum* G. Don sur ladite au moins une poudre de feuilles de *Combretum micranthum* G. Don ;
- le ratio poudre de feuilles de *Combretum micranthum* G. Don / extrait aqueux obtenu à partir de feuilles de *Combretum micranthum* G. Don est de 1:0,5 à 1:2 ;
- ladite douleur est choisie parmi les douleurs articulaires, viscérales, pelviennes, post-opératoires, inflammatoires, neuropathiques, arthritiques ;
- le traitement et/ou la prévention de la douleur est obtenu par l'intermédiaire d'un effet anti-inflammatoire ;
- le traitement et/ou la prévention de la douleur englobe une diminution de son intensité et/ou de sa durée.
- la douleur est choisie parmi les douleurs légères, les douleurs modérées, et les douleurs sévères ;
- ladite préparation contient des kinkeloïdes, avantageusement choisis parmi les kinkeloïdes A, les kinkeloïdes B, les kinkeloïdes C et les kinkeloïdes D.

La présente invention concerne encore une composition pharmaceutique ou non-pharmaceutique, contenant une préparation de *Combretum micranthum* G. Don selon l'invention, pour son utilisation chez des sujets humains ou animaux, dans le traitement et/ou la prévention de la douleur.

De préférence, la composition selon l'invention contient en outre l'un au moins des ingrédients suivants : une préparation d'algue, une préparation de plante, des probiotiques, des vitamines, des minéraux ou tout autre ingrédient nutritionnel.

De préférence, la composition selon l'invention est adaptée à une utilisation dans l'industrie pharmaceutique, cosmétologique, agro-alimentaire, de la nutrition, des compléments alimentaires, de la nutrition animale.

De préférence, la composition est adaptée à une utilisation dans l'industrie cosmétologique, agro-alimentaire, de la nutrition, des compléments alimentaires, de la nutrition animale, dans une utilisation non-thérapeutique d'une préparation de *Combretum micranthum* G. Don, pour son utilisation chez des sujets humains ou animaux, dans le traitement et/ou la prévention de la douleur.

La présente invention concerne encore un procédé pour la fabrication d'une préparation de *Combretum micranthum* G. Don, pour son utilisation chez des sujets humains ou animaux, dans le traitement et/ou la prévention de la douleur.

Ce procédé comprend :
- une étape de fourniture d'au moins une poudre de feuilles de *Combretum micranthum* G. Don, et au moins un extrait aqueux obtenu à partir de feuilles de *Combretum micranthum* G. Don, avantageusement un extrait aqueux concentré, et
- une étape de mélange de ladite au moins une poudre de feuilles de *Combretum micranthum* G. Don et dudit au moins un extrait aqueux obtenu à partir de feuilles de *Combretum micranthum* G. Don.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description des figures

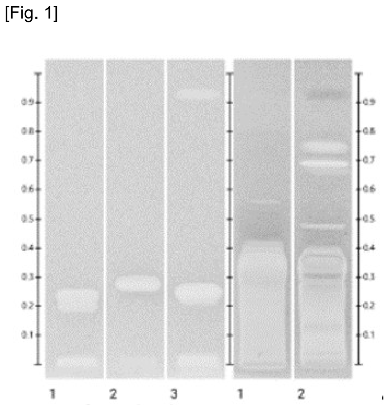

[Fig. 1] est une vue illustrant l'activité biologique étudiée via une inhibition de COX2 en HPTLC.

Légende :
- colonnes de gauche 1 à 3 : Standards kinkéloïdes
- colonne de droite 1 : extrait aqueux
- colonne de droite 2 : préparation selon l'invention

### Description détaillée de l'invention

La présente invention concerne donc, de manière générale, les préparations (et en particulier les extraits) de *Combretum micranthum* G. Don, pour leur utilisation chez des sujets humains ou animaux, dans le traitement et/ou la prévention de la douleur.

Par « sujets humains », on entend en particulier les patients souffrant de douleurs chroniques ou aiguës. Les sujets humains peuvent également inclure des personnes en bonne santé qui souhaitent prévenir les douleurs liées à leur travail, à leur mode de vie, à l'âge, ou les personnes ayant subi une intervention médicale (chirurgicale ou dentaire par exemple) et souffrant de douleurs post-opératoires.

Par « sujets animaux », on entend en particulier les animaux de compagnie tels que les chiens et les chats, qui peuvent souffrir de douleurs chroniques liées à des maladies articulaires ou à d'autres affections, ainsi que les animaux d'élevage tels que les bovins, les porcins, les ovins et les équidés.

Le terme « traitement de la douleur » se réfère à une utilisation pour réduire, soulager ou éliminer la douleur.

Le terme « prévention de la douleur » se réfère à une utilisation pour prévenir ou réduire la douleur avant qu'elle ne se produise.

### Généralité sur les poudres

Dans le cadre de la présente invention et classique en soi, les caractéristiques granulométriques des poudres sont avantageusement déterminées par une technique granulométrique par tamisage.

Le pourcentage en masse représente avantageusement la partie d'une poudre qui reste sur le tamis, dit encore « refus » ou « refus par tamis ».

De telles valeurs peuvent être mesurées selon un protocole décrit par exemple dans le document suivant : Pharmacopée Européenne 9.0, « 2.9.38. Distribution granulométrique par tamisage analytique » ou Pharmacopée Européenne 9.0, « 2.9.35. Finesse des poudres ».

La masse volumique apparente et la masse volumique tassée sont avantageusement obtenues par le procédé décrit dans le document Pharmacopée Européenne 9.7, « 2.9.34. Masse volumique vrac et masse volumique après tassement ».

Les caractéristiques d'une poudre sont par exemple décrites dans le document EP-4 159 198.

De manière générale, selon l'invention, une étape de micronisation est avantageusement mise en œuvre sur le support végétal :
- préalablement à l'étape d'imprégnation, de sorte à ajuster les caractéristiques granulométriques du support végétal avant l'étape d'imprégnation, et/ou
- entre l'étape d'imprégnation et l'étape de séchage, de sorte à ajuster les caractéristiques granulométriques du support végétal imprégné non-séché, et/ou
- postérieurement à l'étape de séchage, de sorte à ajuster les caractéristiques granulométriques du support végétal imprégné et séché.

Encore de manière générale, un procédé de fabrication d'une poudre imprégnée, ainsi que les paramètres caractéristiques d'une poudre, sont décrites par exemple dans le document EP-4 159 198.

### Combretum micranthum G. Don

*Combretum micranthum* ou *Combretum micranthum* G. Don est une espèce d'arbuste non domestiquée que l'on trouve dans la jungle d'Afrique de l'Ouest.

Elle est aussi désignée *Bureava crotonoides, Combretum altum, Combretum floribundum, Combretum parviflorum, Combretum raimbaultii* ou encore kinkéliba (nom vernaculaire).

C'est un arbuste dense ou une liane, que l'on trouve couramment dans les champs cultivés et en jachère, principalement situés en Afrique subsaharienne, avec des rendements plus élevés au Sénégal, au Mali et au Burkina Faso.

Dans plusieurs pays de la savane tropicale d'Afrique de l'Ouest, les feuilles de *Combretum micranthum* sont utilisées comme une tisane traditionnelle populaire, en particulier pour ses propriétés diurétiques.

Encore de manière générale, Combretum micranthum G. Don est encore défini dans la Monographie de la pharmacopée Française, édition 1993.

### Préparation de Combretum micranthum G. Don et ses procédés d'extraction

Par « préparation de plante », on englobe avantageusement les préparations obtenues à partir des matières premières végétales, notamment en les réduisant en poudre ou en les traitant par un procédé d'extraction, de distillation, d'expression, de fractionnement, de purification, de concentration ou de fermentation.

La présente invention concerne en particulier une préparation de *Combretum micranthum* G. Don qui comprend :
- au moins une poudre de feuilles de *Combretum micranthum* G. Don, et
- au moins un extrait aqueux obtenu à partir de feuilles de *Combretum micranthum* G. Don.

Par « poudre de feuilles de *Combretum micranthum* G. Don », on entend avantageusement un produit obtenu par la réduction mécanique des feuilles sèches de *Combretum micranthum* G. Don.

Ce procédé implique la transformation des feuilles séchées en particules solides dont les dimensions sont avantageusement adaptées à des applications pharmaceutiques, cosmétiques ou nutritionnelles.

De préférence, le poudre de feuilles de *Combretum micranthum* G. Don présente les caractéristiques granulométriques suivantes :
- une taille inférieure, ou égale, à 50 µm représentant au maximum 10%, en poids,
- une taille inférieure, ou égale, à 100 µm représentant au maximum 25%, en poids, et
- une taille inférieure, ou égale, à 500µm représentant au minimum 90%, en poids. De préférence encore, la poudre présente encore :
- une masse volumique apparente allant de 0,3 à 0,6 g/cm3, et
- une masse volumique tassée allant de 0,3 à 0,6 g/cm3.

Par « extrait aqueux obtenu à partir de feuilles de *Combretum micranthum G.* Don », on entend avantageusement une préparation liquide issue d'un procédé d'extraction utilisant l'eau comme solvant principal pour isoler les composés bioactifs des feuilles de *Combretum micranthum* G. Don.

Un tel extrait aqueux inclut également les extraits aqueux concentrés.

L'extraction aqueuse est réalisée à partir de feuilles fraîches ou séchées, de préférence séchées, de préférence selon un protocole comprenant :
- une étape de broyage, ou de réduction mécanique, des feuilles pour augmenter la surface d'extraction ;
- une étape de contact entre la matière végétale et l'eau, réalisée à température ambiante ou sous chauffage, par exemple à une température allant de 75°C à 90°C, de préférence pendant une durée de 30 minutes à 2 h (de préférence à l'exclusion d'un mélange de plante et d'eau, chauffé 30mn à 80°C) ;
- une étape de filtration pour séparer l'extrait aqueux des résidus végétaux solides ;
- de préférence une étape de concentration, par exemple sous vide, pour obtenir un extrait aqueux concentré.

Par « extrait aqueux concentré », on entend avantageusement une fraction obtenue par extraction à partir d'une matière végétale puis par concentration de ladite fraction extraite.

La concentration des composés d'intérêt qui sont présents dans l'extrait végétal concentré est supérieure à la concentration des composés d'intérêt qui sont présents dans la matière végétale d'origine.

A cet effet, l'extrait végétal concentré est issu d'une opération d'extraction à partir d'une matière végétale suivie d'une opération de concentration dudit extrait végétal.

De telles opérations d'extraction / concentration sont par exemple décrites dans le document EP-2 080 436 ou dans le document EP-4 159 198.

L'opération de concentration consiste alors avantageusement à concentrer les principes actifs par élimination d'au moins une partie du solvant d'extraction.

La concentration est avantageusement d'un facteur de concentration d'au moins 5, de préférence d'un facteur de concentration de 8 à 15.

Selon un mode de réalisation préféré, ledit au moins un extrait aqueux obtenu à partir de feuilles de *Combretum micranthum* G. Don est imprégné sur ladite au moins une poudre de feuilles de *Combretum micranthum* G. Don.

Par « imprégné », on entend avantageusement un état dans lequel ledit au moins un extrait aqueux obtenu à partir de feuilles de *Combretum micranthum* G. Don est déposé sur la poudre de feuilles de *Combretum micranthum* G. Don, de manière à pénétrer la structure poreuse de la poudre, puis avantageusement séché afin d'éliminer le solvant et d'obtenir une poudre imprégnée stable et homogène.

La préparation selon l'invention se présente avantageusement sous forme d'une poudre imprégnée présentant les caractéristiques granulométriques suivantes :
- une taille inférieure, ou égale, à 50 µm représentant au maximum 10%, en poids,
- une taille inférieure, ou égale, à 100 µm représentant au maximum 25%, en poids, et
- une taille inférieure, ou égale, à 500 µm représentant au minimum 90%, en poids.

De préférence, tel que décrit plus en détails par la suite, la préparation selon l'invention est issue d'un procédé comprenant les étapes suivantes :
- une étape de contact entre des feuilles de *Combretum micranthum* G. Don et un solvant aqueux,
- au moins une étape d'extraction en phase liquide des principes actifs, avantageusement réalisée sous chauffage, par exemple à une température allant de 75°C à 90°C, de préférence pendant 0,5 à 2 h, et/ou sous pression réduite, pour obtenir ledit extrait aqueux contenant lesdits principes actifs,
- de préférence, une étape de concentration dudit extrait aqueux pour obtenir un extrait aqueux concentré, et
- une étape de dépôt dudit au moins un extrait aqueux obtenu à partir de feuilles de *Combretum micranthum* G. Don sur ladite au moins une poudre de feuilles de *Combretum micranthum* G. Don.

De préférence, ladite étape de dépôt consiste en une étape de séchage dudit au moins un extrait aqueux obtenu à partir de feuilles de *Combretum micranthum* G. Don sur ladite au moins une poudre de feuilles de *Combretum micranthum* G. Don.

Encore de manière générale, le ratio massique entre, d'une part, la poudre de feuilles de *Combretum micranthum* G. Don et, d'autre part, l'extrait aqueux obtenu à partir de feuilles de *Combretum micranthum* G. Don, est de préférence de 1:0,5 à 1 :2.

En d'autres termes, ce ratio signifie que la masse d'extrait aqueux représente de 0,5 à 2 fois la masse de la poudre.

De préférence encore et sans être limitatif, le support (poudre) représente de 50% à 90% en poids de la préparation ; l'extrait aqueux représente de 10% à 50% en poids de la préparation.

Encore de manière générale, la préparation de *Combretum micranthum* G. Don selon l'invention contient des kinkeloïdes, composés de la famille des piperidine-flavan alkaloids.

Les kinkeloïdes sont par exemple décrits dans le document Jing Zhen et al., *Total Synthesis of Novel Skeleton Flavan-Alkaloids,* Molecules 2020, 25(19), 4491.

Par « kinkeloïdes », on englobe en particulier les kinkeloïdes A, les kinkeloïdes B, les kinkeloïdes C et les kinkeloïdes D.

Par kinkeloïdes A, on entend les composés chimiques présentant la formule chimique 1 suivantes : 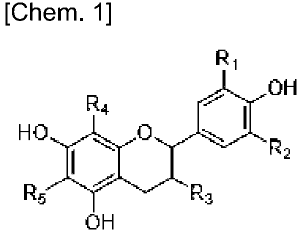 dans laquelle
R₁ est H,
R₂ est H,
R₃ est H,
et dans laquelle :
   R₄ est H et R₅ est pipéridine, ou
   R₄ est pipéridine et R₅ est H.

Par kinkeloïdes B, on entend les composés chimiques présentant la formule chimique 2 suivantes : 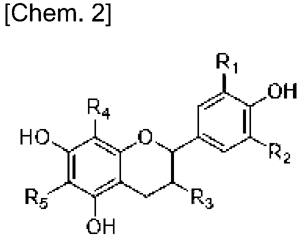 dans laquelle
R₁ est OH,
R₂ est H,
R₃ est H, et
dans laquelle
R₄ est H et R₅ est pipéridine, ou
R₄ est pipéridine et R₅ est H.

Par kinkeloïdes C, on entend les composés chimiques présentant la formule chimique 3 suivantes : 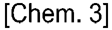 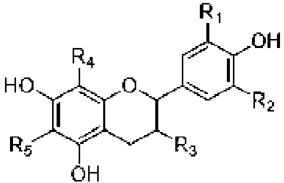 dans laquelle
R₁ est OH
R₂ est OH
R₃ est H,
dans laquelle
R₄ est H et R₅ est pipéridine, ou
R₄ est pipéridine et R₅ est H.

Par kinkeloïdes D, on entend les composés chimiques présentant la formule chimique 4 suivantes : 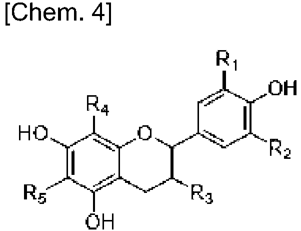 dans laquelle
R₁ est OH
R₂ est OH
R₃ est OH,
dans laquelle
R₄ est H et R₅ est pipéridine, ou
R₄ est pipéridine et R₅ est H.

De préférence, la préparation selon l'invention contient des kinkéloïdes B qui représentent de 70 à 90% des kinkéloïdes totaux.

Sans être limitatif, une posologie préférée pourrait être de 20 mg à 5 g de la préparation selon l'invention, par jour, en une ou plusieurs prises.

### Procédé de fabrication

La présente invention concerne encore un procédé pour la fabrication d'une préparation de *Combretum micranthum* G. Don, pour son utilisation chez des sujets humains ou animaux, dans le traitement et/ou la prévention de la douleur.

Ce procédé comprend :
- une étape de fourniture d'au moins une poudre de feuilles de *Combretum micranthum* G. Don, et au moins un extrait aqueux obtenu à partir de feuilles de *Combretum micranthum* G. Don, avantageusement un extrait aqueux concentré, et
- une étape de mélange de ladite au moins une poudre de feuilles de *Combretum micranthum* G. Don et dudit au moins un extrait aqueux obtenu à partir de feuilles de *Combretum micranthum* G. Don.

De préférence, tel que décrit précédemment, la préparation selon l'invention est issue d'un procédé comprenant les étapes suivantes :
- une étape de contact entre des feuilles de *Combretum micranthum* G. Don et un solvant aqueux,
- au moins une étape d'extraction en phase liquide des principes actifs, pour obtenir ledit extrait aqueux contenant lesdits principes actifs,
- de préférence, une étape de concentration dudit extrait aqueux pour obtenir un extrait aqueux concentré, et
- une étape de dépôt dudit au moins un extrait aqueux obtenu à partir de feuilles de *Combretum micranthum* G. Don sur ladite au moins une poudre de feuilles de *Combretum micranthum* G. Don.

De préférence, ladite au moins une étape d'extraction en phase liquide des principes actifs est :
- réalisée sous chauffage, par exemple à une température allant de 75°C à 90°C,
- de préférence pendant 0,5 à 2 h,
- éventuellement sous pression réduite,
pour obtenir ledit extrait aqueux contenant lesdits principes actifs.

Selon un mode de réalisation préféré, ladite étape de dépôt consiste en une étape de séchage dudit au moins un extrait aqueux obtenu à partir de feuilles de *Combretum micranthum* G. Don sur ladite au moins une poudre de feuilles de *Combretum micranthum* G. Don.

L'imprégnation peut être réalisée par différents procédés, tels que la pulvérisation, le malaxage ou l'enrobage.

Le séchage peut être effectué par différents moyens, tels que le séchage par air chaud, le séchage sous vide ou la lyophilisation.

Le choix du procédé d'imprégnation et de séchage dépendra notamment de la nature de l'extrait concentré, des caractéristiques des poudres et des propriétés souhaitées pour la poudre imprégnée finale.

### Traitement et/ou prévention de la douleur

Par « douleur », on entend en particulier les douleurs articulaires, viscérales, pelviennes, post-opératoires, inflammatoires, neuropathiques, arthritiques.

Le terme « douleur articulaire » se réfère à une douleur ou une gêne ressentie dans une ou plusieurs articulations du corps. Plus particulièrement, l'invention vise à fournir une solution pour le traitement et/ou la prévention des douleurs articulaires, qui peuvent être causées par une variété de facteurs tels que des blessures, des inflammations, des maladies, ou une usure normale liée à l'âge. Les douleurs articulaires peuvent affecter n'importe quelle partie du corps, y compris les mains, les poignets, les coudes, les épaules, les hanches, les genoux, les chevilles et les pieds. Les symptômes peuvent inclure une douleur constante ou intermittente, une raideur, une enflure, une rougeur ou une chaleur autour de l'articulation affectée, ainsi qu'une perte de mobilité et de flexibilité.

Le terme « douleur viscérale » ou « douleur pelvienne » se réfère à une douleur ou une gêne ressentie dans les organes internes du corps, tels que l'estomac, les intestins, la vésicule biliaire, le foie, le pancréas, les reins ou la partie inférieure de l'abdomen. Les douleurs viscérales ou pelviennes peuvent être causées par des affections telles que des ulcères, des infections, des inflammations, des calculs ou des troubles fonctionnels.

Le terme « douleur post-opératoire » se réfère à la douleur et à l'inconfort qui surviennent après une intervention chirurgicale. La douleur post-opératoire est un phénomène courant qui peut varier en intensité et/ou en durée en fonction du type d'intervention, de la sensibilité individuelle et d'autres facteurs. La douleur post-opératoire peut être localisée au site de l'incision ou peut être ressentie dans d'autres parties du corps.

Les douleurs inflammatoires, associées à des phénomènes d'inflammation qui perdurent anormalement, englobent en particulier les douleurs articulaires. Dans ce cas, l'activation chronique des fibres de la douleur entraîne leur sensibilisation qui se généralise ensuite à tout le système de la douleur. Aussi, même en traitant la cause en périphérie, le système peut rester hyper réactif.

Les douleurs neuropathiques sont généralement liées à des atteintes du système nerveux central ou périphérique (lésions de nerfs, blessure, etc.), de la moelle épinière, liées aux amputations ou à un accident vasculaire cérébral. Ces lésions concernent directement le système de détection de la douleur : elles rendent le système d'alarme défaillant et incontrôlable par les antalgiques classiques.

Le terme « douleur arthritique » se réfère à la douleur et à l'inflammation qui surviennent dans les articulations affectées par l'arthrite. L'arthrite est une maladie qui se caractérise par l'inflammation des articulations, qui peut entraîner des douleurs, des raideurs, une réduction de la mobilité articulaire et d'autres symptômes. Les douleurs arthritiques peuvent varier en intensité, en durée et en fréquence en fonction de la gravité de la maladie et de la sensibilité individuelle.

De préférence, la préparation de *Combretum micranthum* G. Don assure le traitement et/ou la prévention de la douleur par l'intermédiaire d'un effet anti-inflammatoire.

De manière générale, par « traitement et/ou prévention de la douleur », la présente invention englobe une diminution de son intensité et/ou de sa durée.

Par « intensité » de la douleur, on englobe la douleur mesurée par des outils pour la caractériser et l'évaluer.

De manière générale, des questionnaires et des échelles de douleur permettent d'en décrire les manifestations, et d'en mesurer l'intensité ainsi que l'impact sur la qualité de vie.

Pour les adultes, l'échelle la plus utilisée est l'échelle numérique ou l'échelle visuelle analogique, graduée de 0 pour une absence de douleur, à 10 pour la douleur maximale imaginable.

Pour les enfants, les médecins utilisent souvent une échelle avec des visages.

Concernant les douleurs neuropathiques, deux échelles permettent respectivement de diagnostiquer ce type de douleurs (DN4) et d'évaluer leur intensité (NPSI).

De manière générale, la présente invention englobe une diminution de l'intensité et/ou de la durée pour la douleur mesurée chez un sujet recevant la préparation de *Combretum micranthum* G. Don selon l'invention par rapport à l'intensité et/ou à la durée pour la douleur mesurée chez un sujet ne recevant pas la préparation de *Combretum micranthum* G. Don selon l'invention.

La douleur englobe en particulier :
- les douleurs légères,
- les douleurs modérées, et
- les douleurs sévères.

Les douleurs légères sont des douleurs peu intenses qui peuvent être facilement supportées par le sujet et n'affectent pas ou peu son activité quotidienne.

Les douleurs modérées sont plus intenses et peuvent affecter le quotidien du sujet, en limitant par exemple ses mouvements ou sa capacité à travailler.

Les douleurs sévères sont les douleurs les plus intenses, qui peuvent être invalidantes et empêcher le sujet de mener une vie normale.

Selon un mode de réalisation préféré, la composition provoque une diminution de l'activité COX-2 / 5-LOX.

De manière générale, l'activité COX-2 / 5-LOX peut être mesurée en utilisant des kits d'essai enzymatique spécifiques, tels que ceux commercialisés par Cayman Chemical.

De manière générale, pour l'activité COX-2, l'essai implique par exemple la conversion de l'acide arachidonique en PGF2, dont la production est quantifiée par une réaction couplée produisant un signal mesurable (colorimétrique ou fluorimétrique). L'ajout de composés inhibiteurs potentiels permet d'évaluer leur effet sur la diminution de la production de PGG2, révélant ainsi leur capacité à inhiber l'enzyme et leur potentiel anti-inflammatoire.

Pour l'activité 5-LOX, l'essai mesure de préférence l'inhibition de l'enzyme 5-lipoxygénase en quantifiant la production de peroxydes lipidiques formés lors de la conversion de l'acide arachidonique en leucotriènes. Cette méthode repose sur une détection colorimétrique qui permet d'évaluer l'efficacité des inhibiteurs dans la réduction de l'activité 5-LOX.

La diminution d'activité est de préférence d'au moins 30%, sans être limitatif.

Ces deux enzymes, la cyclooxygénase-2 (COX-2) et la 5-lipoxygénase (5-LOX), jouent un rôle clé dans la cascade inflammatoire et la production de médiateurs de la douleur, notamment les prostaglandines et les leucotriènes.

En inhibant l'activité de la COX-2 et de la 5-LOX, la composition selon l'invention permet de réduire la production de ces médiateurs inflammatoires et de soulager efficacement la douleur.

Tel que démontré dans les exemples, l'association des différents composants de la composition permet d'obtenir une synergie d'action sur l'inhibition de la COX-2 et de la 5-LOX.

### Composition

La présente invention concerne encore les compositions contenant une préparation de *Combretum micranthum* G. Don selon l'invention, pour son utilisation chez des sujets humains ou animaux, dans le traitement et/ou la prévention de la douleur.

Une telle composition contient avantageusement en outre l'un au moins des ingrédients suivants :
- une préparation d'algue,
- une préparation de plante et
- des probiotiques, y compris des postbiotiques.

La préparation d'algue peut être choisi parmi différentes espèces d'algues, telles que le porphyra, une algue rouge également connue sous le nom de nori, la spiruline, la chlorelle ou une algue brune.

De préférence, la composition selon l'invention comprend une combinaison suivante :
- préparation de *Combretum micranthum* G. Don selon l'invention, et
- préparation de nori.

La préparation de plante peut provenir de différentes plantes, telles que le boswellia, le curcuma, les feuilles de cassis, la saule, la reine des prés, la prêle, l'harpagophytum, la scrofulaire, le cresson du Para (*Acmella oleracea*) ou le gingembre.

De préférence, la composition selon l'invention comprend une combinaison suivante :
- préparation de *Combretum micranthum* G. Don selon l'invention, et
- préparation de gingembre.

De manière générale, par « souche probiotique », on englobe en particulier les microorganismes vivants qui, lorsqu'ils sont ingérés en quantité suffisante, exercent des effets positifs sur la santé, au-delà des effets nutritionnels traditionnels.

La souche probiotique convenant à l'invention est physiologiquement acceptable. En d'autres termes, cette souche probiotique peut être administrée sans risques à l'animal ou l'homme.

Les souches probiotiques englobent notamment les genres lactobacilles (bactéries du genre *Lactobacillus*)*,* bifidobactéries (bactéries du genre *Bifidobacterium*)*,* streptocoques (bactéries du genre *Streptococcus*) ou lactocoques.

Encore de manière générale, des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus, Lactobacillus amylovorus, Lactobacillus casei* (*Shirota*)*, Lactobacillus rhamnosus* (*souche* GG), *Lactobacillus brevis, Lactobacillus crispatus, Lactobacillus delbrueckii* (*subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus salivarius, Lactobacillus alimentarius, Lactobacillus curvatus, Lactobacillus casei subsp. casei, Lactobacillus sake Lactococcus lactis, Enterococcus* (*faecalis, faecium*)*, Lactococcus lactis* (*subspp lactis ou cremoris*)*, Leuconstoc mesenteroides subsp dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces* (*cerevisiae* ou encore *boulardii*)*, Bacillus* (*cereus var toyo ou subtilis*)*, Bacillus coagulans, Bacillus licheniformis, Escherichia coli strain nissle, Propionibacterium freudenreichii* et leurs mélanges.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei ou Bifidobacterium bifidum, Bifidobacterium breve, Bifidobacterium longum, Bifidobacterium animalis, Bifidobacterium lactis, Bifidobacterium infantis, Bifidobacterium adolescentis ou Bifidobacterium pseudocatenulatum* et leurs mélanges.

De manière générale, ladite au moins une souche probiotique est avantageusement choisie parmi les souches probiotiques vivantes ou les souches probiotiques inactivées.

Par forme « vivante », on entend une forme dotée de la capacité à se multiplier sous réserve de la placer dans un environnement propice à la récupération de cette capacité.

Ainsi, au sens de la présente invention, le terme vivant couvre l'état dit dormance dans lequel les microorganismes peuvent être placés suite à un traitement physicochimique tel que par exemple la lyophilisation.

De manière générale, la souche probiotique est avantageusement mise en œuvre sous une forme lyophilisée.

Ce type de formulation possède les avantages d'être facilement accessible, de mise en œuvre aisée et de ne soulever aucune difficulté et/ou contrainte en termes de stockage. Par ailleurs, il est compatible avec le conditionnement de microorganismes à l'état de dormance.

Cette lyophilisation peut être réalisée selon des méthodes conventionnelles ou selon une méthode décrite par exemple dans le document FR-3 103 827.

Par ailleurs, le terme « inactivé » désigne des souches ayant subi un traitement visant à les tuer. De tels traitements peuvent consister, à titre d'exemple non limitatif, en un traitement en autoclave, par ultra-sons, une homogénéisation haute pression ou encore un choc osmotique.

Les souches inactivées sont avantageusement intactes.

La souche probiotique est avantageusement inactivée par la chaleur, dite encore « souche probiotique tyndallisée ».

La concentration en souche probiotique, dans la composition, est par exemple de 10⁷ à 10¹² UFC par dose quotidienne.

La concentration en souche probiotique dans la composition peut être d'une manière générale une quantité efficace permettant d'obtenir l'effet recherché. Cette quantité peut être déterminée par la personne du métier au moyen de ses connaissances générales et par des tests usuels. Cette quantité peut par exemple être, pour une application topique, en dose journalière exprimée en CFU, d'environ 10⁶ à environ 10¹², de préférence au moins 10⁶ à environ 10¹⁰, de préférence d'environ 10⁸ à environ 10⁹. Elle peut être alternativement d'environ 10⁵ à environ 10¹² organismes/g de produit.

La souche probiotique peut encore être incluse à hauteur de de 0,01% à 50% dans la composition, par exemple entre 0,01% et 40%, ou entre 0,01% et 30%, ou entre 0,01% et 10%, ou entre 0,01% et 5%, ou entre 0,01% et 1%, par exemple environ 0,3% (poids/poids).

La composition peut également contenir des postbiotiques, correspondent à des préparations de micro-organismes, avantageusement choisis parmi les souches probiotiques précitées, inanimés et/ou de leurs composants conférant un bénéfice pour la santé de l'hôte.

La composition peut encore contenir d'autres types d'ingrédients, par exemple des vitamines, des minéraux ou tout autre ingrédient nutritionnel.

Plus particulièrement, la composition peut contenir des ingrédients nutraceutiques comme la vitamine C, la glucosamine, la chondroïtine, le palmitoyléthanolamide ou des acides gras.

La composition selon l'invention contient avantageusement une combinaison d'ingrédients adaptés à une utilisation dans l'industrie pharmaceutique, cosmétologique, agro-alimentaire, de la nutrition (y compris les denrées alimentaires destinées à des fins médicales spéciales - DADFMS), des compléments alimentaires, de la nutrition animale.

La composition selon l'invention comprend ainsi avantageusement :
- une préparation de *Combretum micranthum* G. Don selon l'invention, pour son utilisation chez des sujets humains ou animaux, adapté à un traitement et/ou la prévention de la douleur, et
- au moins un support ou un excipient, acceptable en fonction du l'utilisation envisagée.

La préparation de *Combretum micranthum* G. Don de la présente invention peut être préparé sous la forme d'une composition pharmaceutique, qui peut être une formulation telle que des comprimés, des capsules, des poudres, des granules, des solutions, des pastilles, des gelées, des préparations de crème, des sirops, des suspensions, des teintures, des aérosols et autres.

La préparation de *Combretum micranthum* G. Don de la présente invention peut également être préparé sous la forme d'une composition non-pharmaceutique.

La composition non-pharmaceutique englobe les compositions cosmétologiques, les compositions agro-alimentaires, les compositions nutritionnelles, les compléments alimentaires, les compositions pour la nutrition animale.

La composition non-pharmaceutique peut être préparée par des techniques de préparation généralement connues, et des additifs acceptables peuvent être ajoutés à la composition non-pharmaceutique.

Par exemple, la composition non-pharmaceutique selon l'invention consiste avantageusement en un complément alimentaire.

Par « complément alimentaire », on entend avantageusement les compléments alimentaires qui sont soumis à l'ensemble des dispositions générales du droit alimentaire mais aussi aux règles spécifiques définies par la directive 2002/46/CE du Parlement européen et du Conseil du 10 juin 2002 relative au rapprochement des législations des États membres concernant les compléments alimentaires, transposée en droit français par le décret n°2006-352.

Par « compléments alimentaires », on englobe avantageusement les denrées alimentaires dont le but est de compléter le régime alimentaire normal et qui constituent une source concentrée de nutriments ou d'autres substances ayant un effet nutritionnel ou physiologique seuls ou combinés, commercialisés sous forme de doses, à savoir les formes de présentation telles que les ampoules de liquide, les flacons munis d'un compte-gouttes et les autres formes analogues de préparations liquides.

De manière générale, la composition selon l'invention est adaptée à une administration par voie orale, per os ou topique.

### Utilisation non-thérapeutique

La présente invention englobe encore une utilisation non-thérapeutique d'une préparation de *Combretum micranthum* G. Don selon l'invention, pour son utilisation chez des sujets humains ou animaux, dans le traitement et/ou la prévention de la douleur.

Bien entendu, diverses autres modifications peuvent être apportées à l'invention dans le cadre des revendications annexées.

### Exemples

La partie Exemple démontre un effet synergique sur l'inhibition des enzymes COX-2 et 5-LOX à partir de la préparation selon l'invention.

### Activité biologique étudiée via des dosages enzymatiques in tubo

Une approche expérimentale *in tubo* basée sur la mesure de l'activité de la cyclooxygénase-2 (COX-2) et de l'activité de la 5-lipooxygénase (5-LOX), en absence et présence des différents éléments d'essai, a été proposée.

L'activité COX de l'enzyme a été évaluée par la mesure du taux de prostaglandine PGF2a formée après incubation de l'enzyme avec l'acide arachidonique.

L'activité LOX de l'enzyme a été évaluée par la mesure du taux d'hydropéroxydes formés après incubation de l'enzyme avec l'acide linoléique.

**[Tableau 1]**

| | % inhibition COX-2 | |
|---|---|---|
| Fraction aqueuse de *Combretum micranthum* G. Don (69 µg/ml) | | 32 |
| Poudre de *Combretum micranthum G. Don* (220 µg/ml) | | 31 |
| Préparation de *Combretum micranthum* G. Don selon l'invention (289 µg/ml) | | 84 |

**[Tableau 2]**

| | % inhibition 5-LOX | |
|---|---|---|
| Fraction aqueuse de *Combretum micranthum* G. Don (347,37 µg/ml) | | 26 |
| Poudre de *Combretum micranthum G. Don* (1100 µg/ml) | | 58 |
| Préparation de *Combretum micranthum* G. Don selon l'invention (1447,37 µg/ml) | | 100 |

### Activité biologique étudiée via une inhibition de COX2 en HPTLC (Fig. 1).

Les profils montrent que la préparation selon l'invention présente davantage de zones d'inhibition (zones blanches) que l'extrait aqueux, démontrant une différence d'activité sur l'enzyme.

La préparation selon l'invention est ainsi un inhibiteur à la fois de COX2 et de 5 LOX :
- COX2 : effet inhibiteur renforcé pour toutes les doses testées pour la préparation selon l'invention, à quantité égale d'extrait aqueux et/ou de feuilles en poudre ;
- LOX-5 : la composition selon l'invention permet d'augmenter l'effet inhibiteur de l'extrait aqueux.

Ces résultats démontrent que l'invention permet d'obtenir une préparation de plante avec une double activité optimisée.

Ces résultats montrent que la préparation de *Combretum micranthum* G. Don selon l'invention est adaptée au traitement et/ou la prévention de la douleur.

## Revendications

1. Préparation de *Combretum micranthum* G. Don, pour son utilisation chez des sujets humains ou animaux, dans le traitement et/ou la prévention de la douleur, laquelle préparation comprend :
- au moins une poudre de feuilles de *Combretum micranthum* G. Don, et
- au moins un extrait aqueux obtenu à partir de feuilles de *Combretum micranthum G.* Don, avantageusement un extrait aqueux concentré.

2. Préparation de *Combretum micranthum* G. Don pour son utilisation, selon la revendication 1, **caractérisée en ce que** ledit au moins un extrait aqueux obtenu à partir de feuilles de *Combretum micranthum* G. Don est imprégné sur ladite au moins une poudre de feuilles de *Combretum micranthum* G. Don.

3. Préparation de *Combretum micranthum* G. Don pour son utilisation selon la revendication 2, **caractérisée en ce que** ladite préparation se présente sous forme d'une poudre imprégnée présentant les caractéristiques granulométriques suivantes :
- une taille inférieure, ou égale, à 50 µm représentant au maximum 10%, en poids,
- une taille inférieure, ou égale, à 100 µm représentant au maximum 25%, en poids, et
- une taille inférieure, ou égale, à 500µm représentant au minimum 90%, en poids.

4. Préparation de *Combretum micranthum* G. Don pour son utilisation, selon l'une quelconque des revendications 1 à 3, **caractérisée en ce que** ladite préparation est issue d'un procédé comprenant les étapes suivantes :
- une étape de contact entre des feuilles de *Combretum micranthum* G. Don et un solvant aqueux,
- au moins une étape d'extraction en phase liquide des principes actifs, avantageusement réalisée sous chauffage, par exemple à une température allant de 75°C à 90°C, de préférence pendant 0,5 à 2 h, et/ou sous pression réduite, pour obtenir ledit extrait aqueux contenant lesdits principes actifs,
- de préférence, une étape de concentration dudit extrait aqueux pour obtenir un extrait aqueux concentré, et
- une étape de dépôt dudit au moins un extrait aqueux obtenu à partir de feuilles de *Combretum micranthum* G. Don sur ladite au moins une poudre de feuilles de *Combretum micranthum* G. Don.

5. Préparation de *Combretum micranthum* G. Don pour son utilisation, selon la revendication 4, **caractérisée en ce que** ladite étape de dépôt consiste en une étape de séchage dudit au moins un extrait aqueux obtenu à partir de feuilles de *Combretum micranthum* G. Don sur ladite au moins une poudre de feuilles de *Combretum micranthum* G. Don.

6. Préparation de *Combretum micranthum* G. Don pour son utilisation, selon l'une quelconque des revendications 1 à 5, **caractérisée en ce que** le ratio poudre de feuilles de *Combretum micranthum* G. Don / extrait aqueux obtenu à partir de feuilles de *Combretum micranthum* G. Don est de 1 :0,5 à 1:2.

7. Préparation de *Combretum micranthum* G. Don pour son utilisation, selon l'une quelconque des revendications 1 à 6, **caractérisée en ce que** ladite douleur est choisie parmi les douleurs articulaires, viscérales, pelviennes, post-opératoires, inflammatoires, neuropathiques, arthritiques.

8. Composition pharmaceutique ou non-pharmaceutique, contenant une préparation de *Combretum micranthum* G. Don pour son utilisation selon l'une quelconque des revendications 1 à 7, pour son utilisation chez des sujets humains ou animaux, dans le traitement et/ou la prévention de la douleur.

9. Composition selon la revendication 8, contenant en outre l'un au moins des ingrédients suivants : une préparation d'algue, une préparation de plante, des probiotiques, des vitamines, des minéraux ou tout autre ingrédient nutritionnel.

10. Composition selon l'une quelconque des revendications 8 ou 9, adaptée à une utilisation dans l'industrie pharmaceutique, cosmétologique, agro-alimentaire, de la nutrition, des compléments alimentaires, de la nutrition animale.

11. Composition selon la revendication 10, adaptée à une utilisation dans l'industrie cosmétologique, agro-alimentaire, de la nutrition, des compléments alimentaires, de la nutrition animale, dans une utilisation non-thérapeutique d'une préparation de *Combretum micranthum* G. Don, pour son utilisation chez des sujets humains ou animaux, dans le traitement et/ou la prévention de la douleur.

12. Utilisation non-thérapeutique d'une préparation de *Combretum micranthum* G. Don, pour son utilisation chez des sujets humains ou animaux, dans le traitement et/ou la prévention de la douleur,
laquelle préparation comprend :
- au moins une poudre de feuilles de *Combretum micranthum* G. Don, et
- au moins un extrait aqueux obtenu à partir de feuilles de *Combretum micranthum G.* Don, avantageusement un extrait aqueux concentré.

13. Procédé pour la fabrication d'une préparation de *Combretum micranthum* G. Don, pour son utilisation chez des sujets humains ou animaux, dans le traitement et/ou la prévention de la douleur,
lequel procédé comprend :
- une étape de fourniture d'au moins une poudre de feuilles de *Combretum micranthum* G. Don, et au moins un extrait aqueux obtenu à partir de feuilles de *Combretum micranthum* G. Don, avantageusement un extrait aqueux concentré, et
- une étape de mélange de ladite au moins une poudre de feuilles de *Combretum micranthum* G. Don et dudit au moins un extrait aqueux obtenu à partir de feuilles de *Combretum micranthum* G. Don.
